# FASCICULE DE BREVET EUROPEEN

(11) **EP 1 804 863 B1**
(45) Date de publication et mention de la délivrance du brevet: **14.12.2016**
(21) Numéro de dépôt: 05805768.8
(22) Date de dépôt: 20.09.2005
(51) Int. Cl.: A61M 5/20, A61M 5/30

(54) **DISPOSITIF D'INJECTION SANS AIGUILLE FONCTIONNANT AVEC DEUX MATIERES ENERGETIQUES CONCENTRIQUES**
VORRICHTUNG ZUR NADELLOSEN INJEKTION DURCH ZWEI KONZENTRISCHE ENERGETISCHE MATERIALIEN
DEVICE FOR NEEDLELESS INJECTION OPERATING WITH TWO CONCENTRIC ENERGETIC MATERIALS

(30) Priorité: 21.09.2004 FR 0409951
(43) Date de publication de la demande: 11.07.2007
(73) Titulaire: Crossject, 75004 Paris Cédex 04 (FR)
(72) Inventeur: ALEXANDRE, Patrick, 70100 Gray (FR); BROUQUIERES, Bernard, 83100 Toulon (FR); GAUTIER, Philippe, 91220 Le Plessis Pate (FR); REYNAUD, Christiane, 91540 Mennecy (FR)
(74) Mandataire: Gaillarde, Frédéric F. Ch.
(86) Numéro de dépôt international: PCT/FR2005/002325
(87) Numéro de publication internationale: WO 2006/032775

(56) Documents cités:
- EP-A- 0 585 612
- FR-A- 2 807 946
- GB-A- 835 560
- US-A1- 2002 161 329

## Description

Le domaine technique de l'invention est celui des seringues sans aiguille pré-remplies et jetables, fonctionnant avec un générateur de gaz, et utilisées pour les injections intradermiques, sous-cutanées et intramusculaires, de principe actif liquide à usage thérapeutique en médecine humaine ou vétérinaire.

Pour les dispositifs d'injection selon l'invention, un principe actif liquide est constitué par un liquide plus ou moins visqueux, ou un mélange de liquide, ou un gel. Le principe actif peut être un solide mis en solution dans un solvant approprié pour l'injection. Il peut également être représenté par un solide pulvérulent mis en suspension plus ou moins concentré dans un liquide approprié. La granulométrie du principe doit être compatible avec le diamètre des conduits pour éviter les bouchages.

L'utilisation d'une charge pyrotechnique pour ce type de seringue existe déjà et a fait l'objet de plusieurs brevets. A titre d'exemple, on peut citer le brevet US 2,322,244 relatif à un injecteur hypodermique sans aiguille fonctionnant à partir d'une cartouche à blanc. Le liquide à injecter, étant placé au contact de la cartouche, est expulsé de l'injecteur sous l'effet de la pression générée par les gaz de combustion. Un autre brevet, le WO 98/31409, décrit un système d'injection hypodermique impliquant une charge pyrotechnique constituée d'un explosif ou d'une poudre. La spécificité de cet injecteur est qu'il est conçu pour tenter de régler les problèmes liés à la cinétique d'expulsion du principe actif liquide, non pas en jouant sur les caractéristiques de la composition pyrotechnique, mais en présentant une géométrie particulière définissant notamment une chambre annexe d'expansion des gaz munie d'un évent. La charge pyrotechnique, qui se trouve à proximité immédiate du principe actif liquide, agit directement et instantanément sur ledit principe en lui communiquant une vitesse initiale très élevée, tandis que les gaz envahissent la chambre principale et la chambre annexe. La pression exercée sur le principe actif décroît alors pour venir se fixer à une valeur à peu près constante, suffisante pour le faire pénétrer dans la peau du patient. La chambre annexe permet de réguler cette pression. Le brevet US 2,704,542 se rapporte à une méthode d'injection par jet liquide. Cette méthode ne fait pas spécifiquement intervenir une charge pyrotechnique, mais implique un dispositif destiné à maîtriser les profils de pression. En l'occurrence, le procédé mis en place pour atteindre cet objectif réside dans le coulissement, en deux temps, d'un piston en deux parties constitué par un cylindre central de faible section logé dans un cylindre creux. Une pression amont provoque d'abord un déplacement de faible amplitude du cylindre central pour communiquer une impulsion brève mais très intense sur le liquide à expulser, puis l'ensemble du piston se déplace pour continuer à expulser ledit liquide, à la pression idoine, pour assurer une bonne pénétration.

Afin de maîtriser les profils de pression sans avoir à modifier les caractéristiques géométriques de l'injecteur, il a été proposé dans le brevet FR 2 807 946 de réaliser un injecteur sans aiguille muni d'un générateur de gaz impliquant une charge pyrotechnique constituée par le mélange d'une poudre lente avec une poudre rapide. La poudre rapide assure une montée brutale de la pression afin de communiquer au principe actif une vitesse suffisante pour pénétrer à travers la peau et la poudre lente étant choisie pour garantir une profondeur d'injection une fois que la peau a été percée. La demande de brevet américaine US 2002/0161329 évoque également l'utilisation de deux poudres dans un dispositif d'injection lesdites poudres ayant des caractéristiques de combustion différentes. En outre, le brevet GB 835,560 indique l'utilisation de deux poudres pour armes à feu, les deux poudres ayant des caractéristiques de combustion différentes. La demande de brevet européen EP 0 585 612 illustre l'arrière-plan technologique relatif aux générateurs pyrotechniques de gaz.

En revanche, dans l'état de la technique il n'existe pas d'injecteurs sans aiguille qui fassent intervenir une charge pyrotechnique constituée par la combinaison d'une matière énergétique centrale et d'une matière énergétique périphérique ayant des vitesses de combustion différentes. En effet, par ce biais, la charge pyrotechnique qui peut être monolithique ou divisée en plusieurs grains, conserve une grande homogénéité au niveau de la répartition géométrique des deux types de matière énergétiques, conférant ainsi aux dispositifs d'injection selon l'invention un caractère de grande fiabilité et de reproductibilité dans la maîtrise des profils de pression. Cette géométrie particulière de la charge pyrotechnique est issue de procédés de fabrication simples, déjà éprouvés et très bien maîtrisés.

Pour la suite de la description le terme "matière énergétique" regroupe indifféremment toutes les substances pyrotechniques aptes à brûler, que ce soit de la poudre pour arme ou de chasse, du propergol voire même des explosifs.

L'objet de la présente invention concerne un dispositif d'injection sans aiguille comprenant un générateur de gaz pyrotechnique, incluant une charge pyrotechnique constituée de deux matières énergétiques ayant des vitesses de combustion différentes, au moins un piston, une réserve de principe actif liquide et une buse d'injection, l'une des deux matières énergétiques étant centrale et étant entourée par l'autre matière énergétique qui est périphérique, et la totalité de la surface externe de la matière énergétique centrale étant au contact de la surface interne de la matière énergétique périphérique, caractérisé en ce que ladite charge pyrotechnique induit un double régime de combustion entraînant au moins deux phases distinctes au niveau de la variation de pression en sortie de buse d'injection.

Préférentiellement, le générateur de gaz comprend un système d'allumage permettant d'initier en combustion la matière énergétique périphérique, de sorte que le front de combustion évolue de la périphérie de la charge pyrotechnique vers son centre.

Avec une telle charge pyrotechnique et un tel système d'allumage et en fonction des compositions choisies pour les deux matières énergétiques, il peut être envisagée une multiplicité de débits liquides à la sortie de la buse d'injection.

Selon un premier mode de réalisation préféré de l'invention, la matière énergétique périphérique est sélectionnée parmi les compositions qui brûlent très lentement de sorte qu'elle engendre un retard pyrotechnique plus ou moins important pour la combustion de la matière énergétique centrale.

Selon un deuxième mode de réalisation préféré de l'invention, la matière énergétique périphérique est sélectionnée parmi les compositions qui brûlent très rapidement de sorte que la combustion de ladite matière engendre instantanément un pic de pression de forte intensité permettant de favoriser la perforation de la peau.

Les systèmes d'allumage retenus pour les dispositifs d'injection selon l'invention ont des caractéristiques conventionnelles largement éprouvées et peuvent faire intervenir, par exemple, soit un fil chaud parcouru par un courant électrique soit une amorce pouvant être initiée électriquement ou par percussion.

Les deux matières énergétiques ayant des caractéristiques de combustion différentes, le principe de ce type de charge pyrotechnique est d'induire un double régime de combustion entraînant au moins deux phases distinctes au niveau de la variation de pression en sortie de buse d'injection, permettant de résoudre le double problème de la pénétration du principe actif à travers la peau et de la maîtrise de la profondeur d'injection une fois que la peau a été percée.

Avantageusement, la matière énergétique périphérique se trouve à l'état solide de sorte qu'elle constitue une enveloppe pour la matière énergétique centrale.

De façon préférentielle, les deux matières énergétiques sont au contact l'une de l'autre de façon à ce que le front de combustion se transmette d'une matière énergétique à l'autre.

Avantageusement, la totalité de la surface externe de la matière énergétique centrale est au contact de la surface interne de la matière énergétique périphérique.

Selon un premier mode de réalisation préféré de l'invention, la charge pyrotechnique est une poudre composée de plusieurs grains, chaque grain étant constitué d'une matière énergétique centrale entourée par une matière énergétique périphérique, les deux matières énergétiques ayant des vitesses de combustion différentes.

Avantageusement, le générateur de gaz comporte une seule charge pyrotechnique constituée par une poudre, et la matière énergétique périphérique possède une vitesse de combustion supérieure à celle de la matière énergétique centrale. De cette manière, en début de combustion, la pression croît rapidement pour atteindre une valeur seuil, puis une fois que la matière énergétique centrale commence à brûler la pression décroît pour se stabiliser à une valeur prédéterminée inférieure à la valeur seuil ci-avant mentionnée. La pression seuil initiale permet au principe actif de percer la peau du patient à traiter, tandis que la pression obtenue lors de la phase suivante permet au principe actif de pénétrer à travers la peau du patient à une profondeur donnée. Inversement, il peut être choisi une matière énergétique périphérique ayant une vitesse de combustion inférieure à celle de la matière énergétique centrale pour introduire un retard pyrotechnique par rapport à la mise en combustion de la matière énergétique centrale.

Selon un deuxième mode de réalisation préféré de l'invention, le générateur de gaz est composé par un mélange de deux poudres ayant chacune plusieurs grains, l'une étant constituée par une seule matière énergétique, et l'autre étant constituée par une matière énergétique centrale entourée par une matière énergétique périphérique ayant des vitesses de combustion différentes.

De façon avantageuse, la poudre constituée de deux matières énergétiques différentes est obtenue à partir d'un procédé de lissage. Le lissage consiste à faire pénétrer par diffusion un agent chimique qui modifie sur une épaisseur maîtrisée la composition de la matière énergétique, donc son niveau énergétique et sa vitesse de combustion.

Selon un autre mode de réalisation préféré de l'invention, ladite poudre est obtenue à partir d'un procédé d'enrobage. L'enrobage consiste à déposer autour du grain de matière énergétique une pellicule d'épaisseur connue d'un matériau énergétique ou inerte ayant des caractéristiques de combustion maîtrisées comme par exemple, la vitesse de combustion.

Selon un troisième mode de réalisation préféré de l'invention, la charge pyrotechnique est constituée par un bloc monolithique. Ledit bloc peut, par exemple, être cylindrique et posséder un canal central à l'image de la géométrie des blocs de propergol élaborés dans la grosse propulsion.

Selon une autre variante préférée de l'invention, la matière énergétique centrale est à l'état liquide et la matière énergétique périphérique est à l'état solide. La matière énergétique liquide peut, par exemple, être un ergol.

Selon un autre mode de réalisation préféré de l'invention, la matière énergétique centrale est à l'état de gel et la matière énergétique périphérique est à l'état solide. La matière énergétique à l'état de gel peut, par exemple, être également un ergol.

Avantageusement, la charge pyrotechnique constituée d'une matière énergétique centrale à l'état liquide ou de gel et d'une matière énergétique périphérique à l'état solide est obtenue par un procédé d'encapsulage.

L'encapsulage consiste à générer une coque de produit énergétique ou inerte enfermant des matières solides, liquides ou gélifiées.

Selon un autre mode de réalisation préféré de l'invention, la matière énergétique centrale est constituée par au moins une poudre composée d'au moins un grain et la matière énergétique périphérique est constituée par un nitrofilm. En effet, la matière énergétique centrale peut être constituée par une seule poudre ou par le mélange d'au moins deux poudres.

Avantageusement, le nitrofilm comprend un plastifiant, un statibilisant et de la nitrocellulose.

De façon préférentielle, la poudre est une poudre homogène à base de nitrocellulose.

De façon avantageuse, la poudre contient de la nitroglycérine.

Préférentiellement, le nitrofilm constitue une enveloppe fermée pour la poudre.

Le nitrofilm sert, d'une part, de réceptacle à la poudre afin de faciliter son intégration dans le générateur et favorise, d'autre part, l'allumage de la poudre grâce à sa capacité à être lui-même facilement initié en combustion et grâce à sa vitesse de combustion élevée.

Les dispositifs d'injection sans aiguille selon l'invention présentent l'avantage de garantir une injection satisfaisante de la totalité du principe actif liquide, en conservant un mécanisme simple de fonctionnement ainsi qu'un encombrement réduit ne nécessitant, ni la mise en place de pièces spécifiques, sources d'usinages et de coûts supplémentaires, ni une modification en profondeur de la géométrie du corps desdites seringues.

De plus, la grande variabilité des compositions pouvant être retenues pour les charges pyrotechniques incluses dans les dispositifs d'injection selon l'invention, permet d'obtenir une très grande variété de profils de pression aptes à s'adapter à toutes les configurations possibles. Enfin, la parfaite maîtrise des effets engendrés par la combustion d'une charge pyrotechnique associée à des systèmes d'allumage largement éprouvés, confère aux seringues sans aiguille selon l'invention un caractère de grande fiabilité et de sûreté.

La figure 1 est une vue en coupe axiale longitudinale d'un dispositif d'injection sans aiguille selon l'invention.

La figure 2 est un graphe simplifié typique de la variation de pression en sortie de buse en fonction du temps, engendrée par la combustion d'une seule poudre dont la matière énergétique périphérique a une vitesse de combustion inférieure à celle de la matière énergétique centrale.

La figure 3 est un graphe simplifié typique de la variation de pression en sortie de buse en fonction du temps, engendrée par la combustion d'une seule poudre dont la matière énergétique périphérique a une vitesse de combustion supérieure à celle de la matière énergétique centrale.

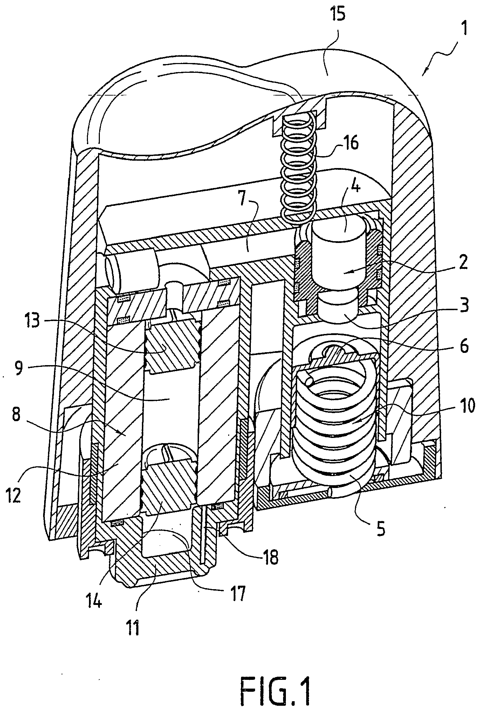

En se référant à la figure 1, un injecteur 1 sans aiguille selon l'invention comprend un générateur de gaz pyrotechnique 2 constitué par une amorce 3 et une charge pyrotechnique 4, un système d'allumage 10 comportant un ressort 5 précontraint et un percuteur 6, une chambre de combustion 7, une colonne de liquide 8 incluant le principe actif liquide 9 à injecter, et une buse d'injection 11. La colonne de liquide 9 comporte un tube en verre 12 fermé par deux bouchons stoppeurs 13, 14 entre lesquels se situe ledit principe actif liquide 9 à injecter. L'injecteur 1 selon l'invention est muni d'un système de déclenchement constitué par un capot 15 et un ressort 16 apte à se comprimer sous l'effet du déplacement dudit capot 15.

Le mode de fonctionnement d'un injecteur sans aiguille 1 selon l'invention est le suivant.

L'injecteur est positionné de façon à ce que la buse 11 vienne en appui contre la peau du patient à traiter.

Une pression sur le capot 15 entraîne le déplacement dudit capot 15 rendu résistant par l'effet du ressort 16 qui se comprime. Au-delà d'un enfoncement seuil dudit capot 15 le système d'allumage 10 est débloqué, entraînant la détente du ressort 5 précontraint et donc le déplacement brutal du percuteur 6 qui lui est associé. Ledit percuteur 6 impacte l'amorce 3 qui est mise en combustion, induisant par influence la combustion de la charge pyrotechnique 4. Les gaz émis par la combustion de ladite charge 4 mettent en pression la chambre de combustion 7 provoquant alors le déplacement de la colonne de liquide 8. Le bouchon piston aval 14 qui est situé le plus près de la buse 11 vient se loger dans un espacement 17 prévu à cet effet, tandis que le bouchon piston amont 13 qui est situé le plus près de la chambre de combustion 7 continue de se déplacer en exerçant une pression sur le liquide à injecter 9. Ledit liquide 9 s'échappe alors par un canal d'injection 18 situé dans la buse 11 et libéré par le bouchon piston 14 aval. L'injection se poursuit jusqu'à ce que le bouchon piston amont 13 vienne au contact du bouchon piston 14 aval.

Les exemples non limitatifs suivants illustrent la caractéristique essentielle de l'invention qui se rapporte à la charge pyrotechnique 4.

### Exemple 1

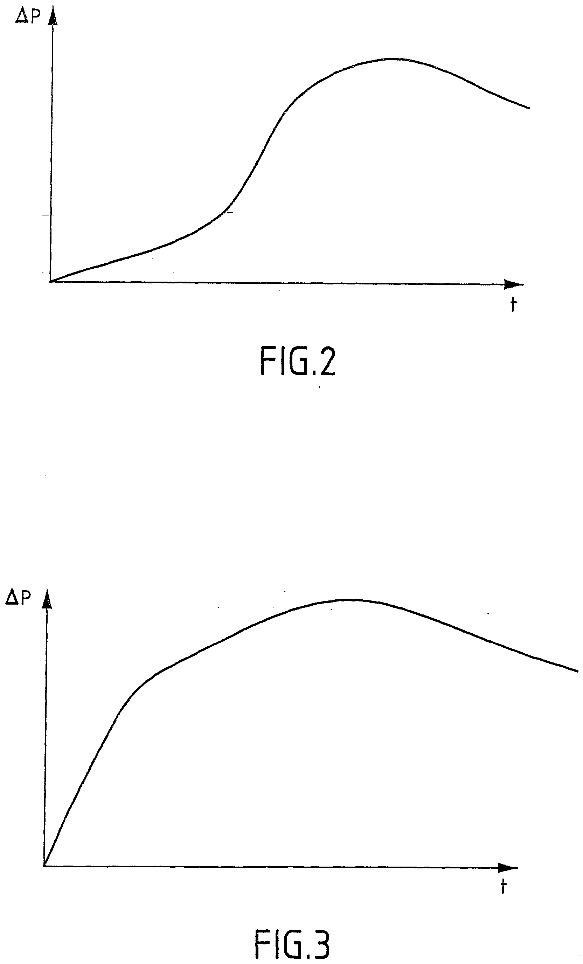

La charge pyrotechnique est constituée par une seule poudre et la matière énergétique périphérique a une vitesse de combustion inférieure à celle de la matière énergétique centrale. Un profil type de pression en fonction du temps obtenu au niveau de la base est représenté à la figure 2. Dans un premier temps, la combustion de la matière énergétique périphérique entraîne une petite augmentation de la pression. Dans un deuxième temps, la pression augmente brutalement due à la combustion de la matière énergétique centrale. Enfin, la pression décroît lentement.

### Exemple 2

La charge pyrotechnique est constituée par une seule poudre et la matière énergétique périphérique a une vitesse de combustion supérieure à celle de la matière énergétique centrale. Un profil type de pression en fonction du temps obtenu au niveau de la buse est représenté à la figure 3. Dans un premier temps, la pression croît brutalement due à la combustion de la matière énergétique périphérique. Dans un deuxième temps, la pression continue d'augmenter plus progressivement et enfin, ladite pression décroît lentement.

Le grand nombre de compositions pyrotechniques déjà existantes pouvant intégrer les dispositifs d'injection sans aiguille selon l'invention permet d'obtenir une grande variabilité au niveau des profils de pression en sortie de buse, permettant ainsi de traiter un grand nombre de configurations.

## Revendications

1. Dispositif d'injection (1) sans aiguille comprenant un générateur de gaz pyrotechnique (2), incluant une charge pyrotechnique constituée de deux matières énergétiques ayant des vitesses de combustion différentes, au moins un piston (13, 14), une réserve de principe actif liquide (9) et une buse d'injection (11), l'une des deux matières énergétiques étant centrale et étant entourée par l'autre matière énergétique qui est périphérique, et la totalité de la surface externe de la matière énergétique centrale étant au contact de la surface interne de la matière énergétique périphérique, **caractérisé en ce que** ladite charge pyrotechnique induit un double régime de combustion entraînant au moins deux phases distinctes au niveau de la variation de pression en sortie de buse d'injection.

2. Dispositif selon la revendication 1 **caractérisé en ce que** la matière énergétique périphérique se trouve à l'état solide.

3. Dispositif selon la revendication 2 **caractérisé en ce que** les deux matières énergétiques sont au contact l'une de l'autre.

4. Dispositif selon la revendication 1 **caractérisé en ce que** la charge pyrotechnique est une poudre composée de plusieurs grains, chaque grain étant constitué d'une matière énergétique centrale entourée par une matière énergétique périphérique, les deux matières énergétiques ayant des vitesses de combustion différentes.

5. Dispositif selon la revendication 4 **caractérisé en ce que** le générateur de gaz (2) comporte une seule charge pyrotechnique constituée par une poudre, et la matière énergétique périphérique a une vitesse de combustion supérieure à celle de la matière énergétique centrale.

6. Dispositif selon la revendication 1 **caractérisé en ce que** le générateur de gaz (2) est composé par un mélange de deux poudres ayant chacune plusieurs grains, l'une étant constituée par une seule matière énergétique et l'autre étant constituée par une matière énergétique centrale entourée par une matière énergétique périphérique ayant des vitesses de combustion différentes.

7. Dispositif selon la revendication 4 **caractérisé en ce que** la poudre est obtenue à partir d'un procédé de lissage.

8. Dispositif selon la revendication 4 **caractérisé en ce que** la poudre est obtenue à partir d'un procédé d'enrobage.

9. Dispositif selon la revendication 1 **caractérisé en ce que** la charge pyrotechnique est constituée par un bloc monolithique.

10. Dispositif selon la revendication 2 **caractérisé en ce que** la matière énergétique centrale est à l'état liquide.

11. Dispositif selon la revendication 2 caractérisé en 25 ce que la matière énergétique centrale est à l'état de gel.

12. Dispositif selon l'une quelconque des revendications 10 ou 11 **caractérisé en ce que** la charge 30 pyrotechnique est obtenue par un procédé d'encapsulage.

13. Dispositif selon la revendication 1 **caractérisé en ce que** la matière énergétique centrale est constituée par au moins une poudre composée d'au moins un grain et la matière énergétique périphérique est constituée par un nitrofilm.

14. Dispositif selon la revendication 13 **caractérisé en ce que** le nitrofilm comprend un plastifiant, un stabilisant et de la nitrocellulose.

15. Dispositif selon la revendication 13 **caractérisé en ce que** la poudre est une poudre homogène à base de nitrocellulose.

16. Dispositif selon la revendication 15 caractérisé en 10 ce que la poudre contient de la nitroglycérine.

17. Dispositif selon la revendication 13 **caractérisé en ce que** le nitrofilm constitue une enveloppe fermée pour la poudre.

18. Dispositif selon la revendication 1 **caractérisé en ce que** le générateur de gaz (2) comprend un système d'allumage (10) permettant d'initier en combustion la matière énergétique périphérique.

## Patentansprüche

1. Nadellose Injektionsvorrichtung (1), welche einen pyrotechnischen Gasgenerator (2) umfasst, der eine pyrotechnische Ladung bestehend aus zwei Energieträgern mit unterschiedlichen Verbrennungsgeschwindigkeiten, mindestens einen Kolben (13, 14), eine Reserve an flüssigem Wirkstoff (9) und eine Injektionsdüse (11) einschließt, wobei einer der beiden Energieträger mittig ist und von dem anderen Energieträger, der umlaufend ist, umschlossen ist, und die Gesamtheit der Außenfläche des mittigen Energieträgers mit der Innenfläche des umlaufenden Energieträgers in Kontakt ist, **dadurch gekennzeichnet, dass** die besagte pyrotechnische Ladung ein zweifaches Verbrennungsregime induziert, das im Hinblick auf die Druckänderung am Ausgang der Injektionsdüse mindestens zwei verschiedene Phasen bewirkt.

2. Vorrichtung nach Anspruch 1, **dadurch gekennzeichnet, dass** der umlaufende Energieträger in festem Zustand vorliegt.

3. Vorrichtung nach Anspruch 2, **dadurch gekennzeichnet, dass** die zwei Energieträger miteinander in Kontakt sind.

4. Vorrichtung nach Anspruch 1, **dadurch gekennzeichnet, dass** die pyrotechnische Ladung ein Pulver ist, das aus mehreren Körnchen zusammengesetzt ist, wobei jedes Körnchen aus einem mittigen, von einem umlaufenden Energieträger umschlossenen Energieträger besteht, wobei die zwei Energieträger unterschiedliche Verbrennungsgeschwindigkeiten besitzen.

5. Vorrichtung nach Anspruch 4, **dadurch gekennzeichnet, dass** der Gasgenerator (2) eine einzige pyrotechnische Ladung umfasst, die aus einem Pulver besteht, und der umlaufende Energieträger eine Verbrennungsgeschwindigkeit höher jener des mittigen Energieträgers besitzt.

6. Vorrichtung nach Anspruch 1, **dadurch gekennzeichnet, dass** der Gasgenerator (2) aus einer Mischung aus zwei Pulvern mit jeweils mehreren Körnchen zusammengesetzt ist, wobei das eine aus einem einzigen Energieträger besteht und das andere aus einem mittigen, von einem umlaufenden Energieträger umschlossenen Energieträger besteht, welche unterschiedliche Verbrennungsgeschwindigkeiten besitzen.

7. Vorrichtung nach Anspruch 4, **dadurch gekennzeichnet, dass** das Pulver mittels eines Glättungsverfahrens hergestellt wird.

8. Vorrichtung nach Anspruch 4, **dadurch gekennzeichnet, dass** das Pulver mittels eines Umhüllungsverfahrens hergestellt wird.

9. Vorrichtung nach Anspruch 1, **dadurch gekennzeichnet, dass** die pyrotechnische Ladung aus einem monolithischen Block besteht.

10. Vorrichtung nach Anspruch 2, **dadurch gekennzeichnet, dass** der mittige Energieträger in flüssigem Zustand vorliegt.

11. Vorrichtung nach Anspruch 2, **dadurch gekennzeichnet**, 25 dass der mittige Energieträger in gelförmigem Zustand vorliegt.

12. Vorrichtung nach einem der Ansprüche 10 oder 11, **dadurch gekennzeichnet, dass** die pyrotechnische Ladung 30 mittels eines Verkapselungsverfahrens hergestellt wird.

13. Vorrichtung nach Anspruch 1, **dadurch gekennzeichnet, dass** der mittige Energieträger aus mindestens einem Pulver besteht, das aus mindestens einem Körnchen zusammengesetzt ist, und der umlaufende Energieträger aus einem Nitrofilm besteht.

14. Vorrichtung nach Anspruch 13, **dadurch gekennzeichnet, dass** der Nitrofilm einen Weichmacher, einen Stabilisator und Nitrozellulose umfasst.

15. Vorrichtung nach Anspruch 13, **dadurch gekennzeichnet, dass** das Pulver ein homogenes Pulver auf Basis von Nitrozellulose ist.

16. Vorrichtung nach Anspruch 15, **dadurch gekennzeichnet**, 10 dass das Pulver Nitroglycerin enthält.

17. Vorrichtung nach Anspruch 13, **dadurch gekennzeichnet, dass** der Nitrofilm eine geschlossene Hülle für das Pulver bildet.

18. Vorrichtung nach Anspruch 1, **dadurch gekennzeichnet, dass** der Gasgenerator (2) ein Zündsystem (10) umfasst, welches es ermöglicht, den umlaufenden Energieträger in Brand zu setzen.

## Claims

1. An injection device (1) without needle comprising a pyrotechnic gas generator (2), including a pyrotechnic charge constituted of two energetic materials having different combustion rates, at least one piston (13, 14), a liquid active principle reservoir (9) and an injection nozzle (11), one of the two energetic materials being central and being surrounded by the other energetic material which is peripheral, and the entire external surface of the central energetic material being in contact with the internal surface of the peripheral energetic material, **characterized in that** said pyrotechnic charge induces a dual-regime of combustion driving at least two distinct phases at the pressure variation at the outlet of the injection nozzle.

2. The device according to claim 1 **characterized in that** the peripheral energetic material is in the solid state.

3. The device according to claim 2 **characterized in that** the two energetic materials are in contact with each other.

4. The device according to claim 1 **characterized in that** the pyrotechnic charge is a powder composed of several grains, each grain being constituted of a central energetic material surrounded by a peripheral energetic material, the two energetic materials having different combustion rates.

5. The device according to claim 4 **characterized in that** the gas generator (2) includes a single pyrotechnic charge constituted of a powder, and the peripheral energetic material has a combustion rate higher than the combustion rate of the central energetic material.

6. The device according to claim 1 **characterized in that** the gas generator (2) is composed by a mixture of two powders each having several grains, one being constituted by a single energetic material and the other being constituted by a central energetic material surrounded by a peripheral energetic material having different combustion rates.

7. The device according to claim 4 **characterized in that** the powder is obtained from a smoothing method.

8. The device according to claim 4 **characterized in that** the powder is obtained from a coating method.

9. The device according to claim 1 **characterized in that** the pyrotechnic charge is constituted by a monolithic block.

10. The device according to claim 2 **characterized in that** the central energetic material is in the liquid state.

11. The device according to claim 2 **characterized in that** the central energetic material is in the gel state.

12. The device according to any one of claims 10 or 11 **characterized in that** the pyrotechnic charge 30 is obtained by an encapsulation method.

13. The device according to claim 1 **characterized in that** the central energetic material is constituted by at least one powder composed of at least one grain and the peripheral energetic material is constituted by a nitrofilm.

14. The device according to claim 13 **characterized in that** the nitrofilm comprises a plasticizer, a stabilizer and nitrocellulose.

15. The device according to claim 13 **characterized in that** the powder is a nitrocellulose based homogeneous powder.

16. The device according to claim 15 **characterized in that** the powder contains nitroglycerine.

17. The device according to claim 13 **characterized in that** the nitrofilm constitutes a closed envelope for the powder.

18. The device according to claim 1 **characterized in that** the gas generator (2) comprises an ignition system (10) allowing to initiate the peripheral energetic material into combustion.
